# EUROPEAN PATENT SPECIFICATION

(11) **EP 4 299 567 B1**
(45) Date of publication and mention of the grant of the patent: **29.10.2025**
(21) Application number: 22759705.1
(22) Date of filing: 24.02.2022
(51) Int. Cl.: C07D 307/68, C07B 61/00

(54) **METHOD FOR PRODUCING 2-FURONITRILE AND METHOD FOR PRODUCING CARBONIC ACID ESTER**
VERFAHREN ZUR HERSTELLUNG VON 2-FURONITRIL UND VERFAHREN ZUR HERSTELLUNG VON KOHLENSÄUREESTER
PROCÉDÉ DE PRODUCTION DE 2-FURONITRILE ET PROCÉDÉ DE PRODUCTION D'UN ESTER DE L'ACIDE CARBONIQUE

(30) Priority: 25.02.2021 JP 2021028058
(43) Date of publication of application: 03.01.2024
(73) Proprietor: MITSUBISHI GAS CHEMICAL COMPANY, INC., Chiyoda-ku Tokyo 100-8324 (JP); NIPPON STEEL CORPORATION, Chiyoda-ku Tokyo 100-8071 (JP); Nippon Steel Engineering Co., Ltd., Tokyo 141-8604 (JP)
(72) Inventor: TOMISHIGE Keiichi, Sendai-shi, Miyagi 980-8577 (JP); NAKAGAWA Yoshinao, Sendai-shi, Miyagi 980-8577 (JP); YABUSHITA Mizuho, Sendai-shi, Miyagi 980-8577 (JP); SUZUKI Kimihito, Tokyo 100-8071 (JP); KATO Yuzuru, Tokyo 141-8604 (JP); MORITA Kentaro, Tokyo 141-8604 (JP); HARADA Hidefumi, Tokyo 125-8601 (JP); SHINKAI Yousuke, Tokyo 125-8601 (JP); UMEZU Ryotaro, Tokyo 125-8601 (JP)
(74) Representative: Hoffmann Eitle
(86) International application number: PCT/JP2022/007538
(87) International publication number: WO 2022/181670

(56) References cited:
- WO-A1-2015/099053
- JACQUELINE CAMPBELL ET AL: "Laboratory-Scale Synthesis of Nitriles by Catalysed Dehydration of Amides and Oximes under Flash Vacuum Pyrolysis (FVP) Conditions", SYNTHESIS, vol. 2007, no. 20, 1 October 2007 (2007-10-01), STUTTGART, DE., pages 3179 - 3184, XP055494863, ISSN: 0039-7881, DOI: 10.1055/s-2007-990782
- MA XIAOYUN ET AL: "Efficient Mo(VI)-Catalyzed Hydration of Nitrile with Acetaldoxime", vol. 44, no. 4, 16 February 2014 (2014-02-16), US, pages 474 - 480, XP093177219, ISSN: 0039-7911, Retrieved from the Internet <URL:https://www.tandfonline.com/doi/epdf/10.1080/00397911.2013.806668?needAccess=true> DOI: 10.1080/00397911.2013.806668
- NAGASAKI YO-HEI ET AL: "Dehydration of Amides to Nitriles over Heterogeneous Silica-Supported Molybdenum Oxide Catalyst", vol. 14, no. 9, 8 March 2022 (2022-03-08), Hoboken, USA, XP055961315, ISSN: 1867-3880, Retrieved from the Internet <URL:https://onlinelibrary.wiley.com/doi/full-xml/10.1002/cctc.202101846> DOI: 10.1002/cctc.202101846
- NAGASAKI YO‐HEI, TAMURA MASAZUMI, YABUSHITA MIZUHO, NAKAGAWA YOSHINAO, TOMISHIGE KEIICHI: "Dehydration of Amides to Nitriles over Heterogeneous Silica‐Supported Molybdenum Oxide Catalyst", CHEMCATCHEM, vol. 14, no. 9, 6 May 2022 (2022-05-06), XP055961315, ISSN: 1867-3880, DOI: 10.1002/cctc.202101846
- GANESAN MUTHUPANDIAN, NAGARAAJ PARAMATHEVAR: "Recent developments in dehydration of primary amides to nitriles", ORGANIC CHEMISTRY FRONTIERS, vol. 7, no. 22, 10 November 2020 (2020-11-10), pages 3792 - 3814, XP055961316, DOI: 10.1039/D0QO00843E
- AL‐HUNITI MOHAMMED H., CROATT MITCHELL P.: "Metal‐Catalyzed Dehydration of Primary Amides to Nitriles", ASIAN JOURNAL OF ORGANIC CHEMISTRY , 1(2), 160-165 CODEN: AJOCC7; ISSN: 2193-5807, WILEY - V C H VERLAG GMBH & CO. KGAA, GERMANY, vol. 8, no. 10, 1 October 2019 (2019-10-01), Germany , pages 1791 - 1799, XP055961318, ISSN: 2193-5807, DOI: 10.1002/ajoc.201900343
- SHIPILOVSKIKH SERGEI A., VAGANOV VLADIMIR YU., DENISOVA ELENA I., RUBTSOV ALEKSANDR E., MALKOV ANDREI V.: "Dehydration of Amides to Nitriles under Conditions of a Catalytic Appel Reaction", ORGANIC LETTERS, AMERICAN CHEMICAL SOCIETY, US, vol. 20, no. 3, 2 February 2018 (2018-02-02), US , pages 728 - 731, XP055961320, ISSN: 1523-7060, DOI: 10.1021/acs.orglett.7b03862

## Description

### Technical Field

The present invention relates to a method for producing 2-furonitrile and a method for producing a carbonic acid ester.

### Background Art

Carbonic acid ester is a general term for compounds obtained by replacing one or two of two hydrogen atoms of carbonic acid, CO(OH)₂, with alkyl groups or aryl groups, and has a structure represented by RO-C(=O)-OR' (R and R' each represent a saturated hydrocarbon group or an unsaturated hydrocarbon group).

Carbonic acid esters are very useful compounds for use as additives such as gasoline additives for enhancing the octane number and diesel fuel additives for reducing particles in exhaust gas; alkylating agents, carbonylating agents, solvents or the like for synthesizing resins and organic compounds such as polycarbonates, urethanes, pharmaceutical agents, and agricultural chemicals; electrolytes for lithium ion batteries; lubricant oil raw materials; and raw materials of deoxidizers for preventing corrosion of boiler pipes.

A conventional method mainly used for producing a carbonic acid ester is a method in which phosgene as a carbonyl source is directly reacted with an alcohol. Since this method uses phosgene that is very harmful and highly corrosive, it is necessary to take great care to handle it during transport and storage and it takes huge costs to operate and maintain production facilities and guarantee safety. Further, when a carbonic acid ester is produced by this method, a halogen such as chlorine is contained in a raw material and a catalyst, and therefore the resulting carbonic acid ester contains a trace amount of halogen that cannot be removed by a simple purification process. When used for gasoline additives, light oil additives, and electronic materials, such a carbonic acid ester is required to be subjected to a thorough purification process to reduce the trace amount of halogen contained therein to the level of an extremely trace amount because the halogen may cause corrosion. Further, due to recent strict administrative guidance, new production facilities for this method using phosgene that is very harmful to humans cannot be constructed. For this reason, there has been a strong demand for development of a novel method for producing a carbonic acid ester without using phosgene.

Also, there is a known method for directly synthesizing a carbonic acid ester from an alcohol and carbon dioxide using a heterogeneous catalyst. This method uses 2-cyanopyridine or benzonitrile as a dehydrant to increase an amount of a carbonic acid ester produced and is intended to greatly improve the amount and speed of production of a carbonic acid ester, to allow the reaction to easily proceed under a pressure close to ordinary pressure, and to increase the speed of the reaction (see Patent Literatures 1 and 2). However, this method still has some points that need to be improved in terms of treatment and utilization of benzamide or the like generated as a by-product.

For example, the use of benzamide produced by a reaction between benzonitrile and water is limited to some pharmaceutical and agricultural intermediates. Therefore, in production of a carbonic acid ester using benzonitrile as a dehydrant, it is desirable to regenerate benzonitrile from benzamide generated as a by-product and reuse the regenerated benzonitrile. However, a problem still remains in that it is necessary to perform this regeneration reaction highly selectively (because reuse as a dehydrant is considered to be difficult when a by-product is generated) in a high yield (because a low yield increases the amount of residual benzamide, that is, the amount of benzamide that should be separated so that the load of separating it from benzonitrile increases).

In view of the fact that, as described above, there are some points that need to be improved in terms of regeneration of benzonitrile or the like from benzamide or the like, there is a known method to perform the regeneration described above without using a strong reagent while reducing the generation of a by-product (Patent Literature 3).

However, this method also has some points that need to be improved: one is that this method cannot be used in combination with a carbonic acid ester synthesis reaction because it takes 400 hours to produce (regenerate) a nitrile by dehydration of an amide compound, which is not balanced with the carbonic acid ester synthesis reaction that is completed in 24 hours, and the other is that extraction and filtration are required to remove a catalyst by liquid-solid separation, which elongates and complicates the process.

### Citation List

### Patent Literatures

Patent Literature 1: Japanese Patent Laid-Open No. 2010-77113
Patent Literature 2: Japanese Patent Laid-Open No. 2012-162523
Patent Literature 3: International Publication No. WO 2015/099053
Jacqueline Campbell et al.: "Laboratory-Scale Synthesis of Nitriles by Catalysed Dehydration of Amides and Oximes under Flash Vacuum Pyrolysis (FVP) Conditions", SYNTHESIS, vol. 2007, no. 20, 1 October 2007, pages 3179-3184

### Summary of Invention

### Technical Problem

In view of the above problems of the conventional art, it is an object of the present invention to provide a method that makes it possible to achieve a dehydration reaction by which a target compound can selectively be obtained in a high yield while reducing the generation of a by-product in regeneration of a nitrile by dehydration of an amide compound.

It is also an object of the present invention to achieve a method for efficiently producing a carbonic acid ester by applying the above method for producing (regenerating) a nitrile compound to a method for producing a carbonic acid ester.

### Solution to Problem

In order to achieve the above objects, the present inventor has intensively studied a method for producing a nitrile compound by dehydration of an amide compound and as a result has found that 2-furonitrile as a target compound can selectively be obtained in a high yield while the generation of a by-product is reduced by dehydrating 2-furamide in the presence of a specific catalyst.

This finding has made it possible to balance the speed of regeneration of a nitrile compound from an amide compound by a dehydration reaction and the speed of synthesis of a carbonic acid ester from CO₂ and an alcohol using a nitrile compound, that is, to establish the dehydration reaction and the carbonic acid ester synthesis reaction as a set of commercial processes. Therefore, the present inventor has further studied the application of this finding to a method for producing a carbonic acid ester.

More specifically, the present invention provides the following aspects.
<1> A method for producing 2-furonitrile, comprising dehydrating 2-furamide in the presence of an Mo/SiO₂ catalyst in which molybdenum (Mo) is supported on a carrier formed from SiO₂. 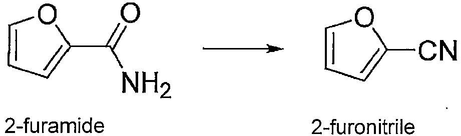
<2> The method for producing 2-furonitrile according to the above <1>, wherein the dehydration is further performed in the presence of a desiccant.
<3> The method for producing 2-furonitrile according to the above <2>, wherein the desiccant is a molecular sieve.
<4> The method for producing 2-furonitrile according to any one of the above <1> to <3>, wherein 2-furancarboxylic acid is produced together with the 2-furonitrile. 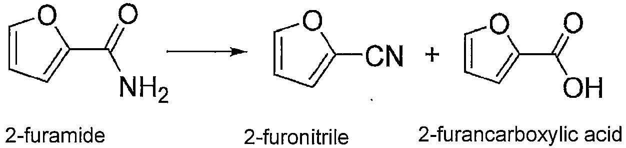
<5> A method for producing a carbonic acid ester, comprising:
   a first reaction step including a carbonic acid ester production reaction in which an alcohol and carbon dioxide are reacted in the presence of a solid catalyst, 2-furonitrile, and a solvent to produce a carbonic acid ester and water and a hydration reaction in which the 2-furonitrile is hydrated with the produced water to produce 2-furamide; and
   a second reaction step in which the 2-furamide is separated from a reaction system of the first reaction step, and then dehydration of the 2-furamide is performed in the presence of an Mo/SiO₂ catalyst in which molybdenum (Mo) is supported on a carrier formed from SiO₂ to regenerate 2-furonitrile, wherein
   at least a part of the 2-furonitrile regenerated in the second reaction step is used in the first reaction step.
<6> The method for producing a carbonic acid ester according to the above <5>, wherein the dehydration is further performed in the presence of a desiccant.
<7> The method for producing a carbonic acid ester according to the above <6>, wherein the desiccant is a molecular sieve.
<8> The method for producing a carbonic acid ester according to any one of the above <5> to <7>, wherein the alcohol comprises an alcohol having 1 to 6 carbon atoms.
<9> The method for producing a carbonic acid ester according to any one of the above <5> to <8>, wherein the solid catalyst comprises at least one selected from CeO₂ and ZrO₂.
<10> The method for producing a carbonic acid ester according to any one of the above <5> to <9>, wherein in the first reaction step, a solvent having a boiling point higher than that of the 2-furamide to be produced is used.
<11> The method for producing a carbonic acid ester according to the above <10>, wherein the solvent comprises at least one selected from dialkylbenzene, alkylnaphthalene, and diphenylbenzene.

### Advantageous Effects of Invention

As described above, the present invention makes it possible to efficiently produce (regenerate) a nitrile compound from an amide compound. Specifically, in a dehydration reaction of an amide compound for the regeneration descried above, it is possible to selectively obtain a target compound in a high yield while reducing the generation of a by-product. Therefore, the present invention makes it possible to greatly reduce the reaction time of a dehydration reaction for regenerating a nitrile compound as compared to the conventional method.

Further, the present invention also makes it possible to achieve a method for efficiently producing a carbonic acid ester by producing a nitrile compound in such a manner as described above.

### Brief Description of Drawing

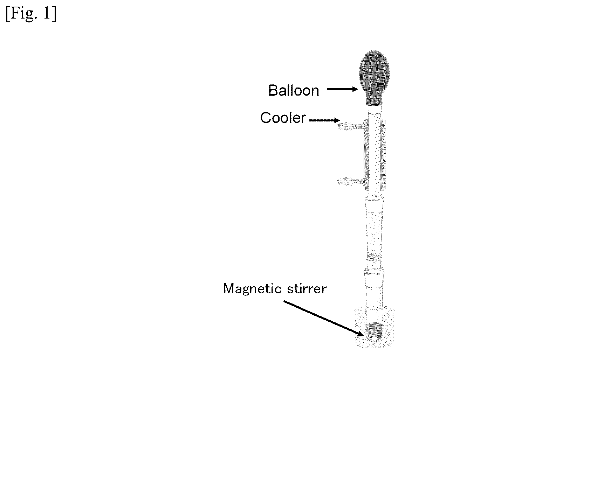

[Figure 1] Figure 1 is a schematic diagram showing a reactor (furnace) used in Examples and Comparative Examples.

### Description of Embodiments

Hereinbelow, preferred embodiments of the present invention will be described in detail.

### <Method for producing 2-furonitrile>

A method for producing 2-furonitrile according to an embodiment of the present invention is a method in which dehydration of 2-furamide is performed in the presence of a Mo/SiO₂ catalyst obtained by supporting molybdenum (Mo) on a carrier made of SiO₂ to produce 2-furonitrile.

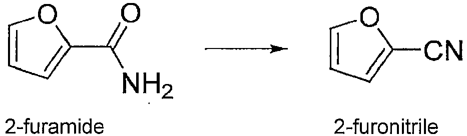

The catalyst used in the above dehydration reaction according to the present invention is produced by, for example, the following method. As the carrier, a commercially-available SiO₂ powder or commercially-available SiO₂ spherical particles can be used. In order to uniformly support molybdenum (Mo) as an active metal, SiO₂ is preferably sized 100 mesh (0.15 mm) or less and subjected to preliminary firing in air at 700°C for 1 hour to remove moisture. There are various types of SiO₂ different in properties, but SiO₂ having a large surface area is preferred because molybdenum (Mo) can highly be dispersed so that an amount of 2-furonitrile produced increases. Specifically, a surface area of 300 m²/g or more is preferred. It should be noted that there is a case where the surface area of the prepared catalyst is lower than that of SiO₂ alone due to, for example, interaction between SiO₂ and molybdenum (Mo). In this case, the surface area of the produced catalyst is preferably 150 m²/g or more. Molybdenum (Mo) that acts as an active species can be supported by an impregnation method such as an incipient wetness method or an evaporation-to-dryness method.

A metal salt as the catalyst precursor is not particularly limited as long as it is water-soluble, and various compounds can be used. The catalyst to be used can be prepared by impregnating the carrier with an aqueous precursor solution of a basic metal and then drying and firing the resultant. The temperature of firing depends on the precursor to be used but is preferably 400 to 600°C.

The catalyst loading may appropriately be set, but for example, the loading of molybdenum (Mo) oxide in terms of metal with respect to the total weight of the catalyst is preferably set to about 0.1 to 1.5 mmol/g and particularly preferably set to about 0.1 to 1 mmol/g. If the loading is more than the above upper limit, the activity may reduce.

The amount of the catalyst used in the reaction may appropriately be set but is preferably 20 to 150 parts by mass, more preferably 30 to 130 parts by mass per 100 parts by mass of 2-furamide used.

The catalyst used in the present invention is preferably one obtained by supporting only a molybdenum (Mo) oxide on a carrier made of SiO₂, but may contain, in addition to the above elements, inevitable impurities incorporated during, for example, a catalyst production process. However, it is desirable that incorporation of impurities is prevented as much as possible.

The catalyst used in the present invention which is obtained by supporting a molybdenum (Mo) oxide that acts as an active species on a carrier may be in the form of either powder or a shaped form. In the case of a shaped form, the catalyst may have any shape such as a spherical shape, a pellet-like shape, a cylindrical shape, a ring shape, a wheel-like shape, or a granular shape.

In the method for producing 2-furonitrile using a catalyst according to the present invention, the type of the reaction is not particularly limited, and any reactor such as a batch reactor, a semibatch reactor, or a flow reactor such as a continuous stirred tank reactor or a piston flow reactor may be used. The catalyst can be applied to any of a fixed-bed reactor, a slurry-bed reactor or the like.

In the method for producing 2-furonitrile according to the present invention, the reaction can usually be performed under conditions of a reaction liquid temperature of 160 to 230°C, a pressure of ordinary pressure (101.3 (kPa) (760 Torr)) to reduced pressure (1.33 (kPa) (10 Torr)), and a time of about several hours to 100 hours, but the reaction conditions are not particularly limited thereto.

For example, the reaction liquid temperature is preferably 170 to 220°C, more preferably 180 to 210°C. The reaction time is preferably 6 to 90 hours, more preferably 10 to 80 hours, particularly preferably 20 to 75 hours. The pressure is preferably ordinary pressure from the viewpoint of lack of the need for a decompressor and energy efficiency.

In the method for producing 2-furonitrile according to the present invention, the dehydration of 2-furamide is preferably performed in the presence of a desiccant in addition to the Mo/SiO₂ catalyst. The type of the desiccant is not particularly limited, but a molecular sieve is preferably used.

When a molecular sieve is used as the dehydrating agent, the type and shape of the molecular sieve are not particularly limited, but for example, a spherical or pellet-shaped molecular sieve generally having high water absorbability, such as type 3A, 4A, or 5A can be used. For example, Zeolum manufactured by Tosoh Corporation can suitably be used. Further, the molecular sieve is preferably beforehand dried at 300 to 500°C for about 1 hour.

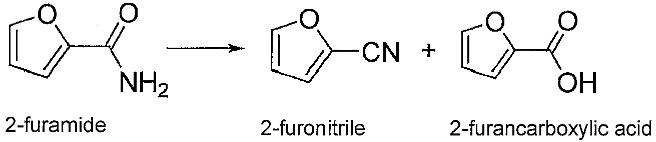

In the present invention, there is a case where, as shown above, 2-furancarboxylic acid is generated as a by-product by decomposition of 2-furamide in the dehydration reaction of 2-furamide. However, a reaction liquid after the dehydration reaction using the reaction conditions according to the present invention contains large amounts of unreacted 2-furamide and 2-furonitrile as a product, and the by-product shown in the above formula is less likely to be generated.

### <Method for producing carbonic acid ester using 2-furonitrile>

As described above, in regeneration of 2-furonitrile from 2-furamide by a dehydration reaction, the target compound could selectively be obtained in a high yield without using a strong reagent while generation of a by-product was reduced. This has made it possible to balance the speed of regeneration of 2-furonitrile from 2-furamide by a dehydration reaction and the speed of synthesis of a carbonic acid ester from CO₂ and an alcohol using 2-furonitrile, that is, to combine the dehydration reaction and the carbonic acid ester synthesis reaction to establish these reactions as a set of commercial processes. Therefore, the present inventor has applied this finding to a method for producing a carbonic acid ester, which has made it possible to arrive at a method for producing a carbonic acid ester described below.

### (First reaction step)

A first reaction step in a method for producing a carbonic acid ester according to the present invention includes a reaction (carbonic acid ester production reaction) in which an alcohol and carbon dioxide are directly reacted in the presence of a solid catalyst such as CeO₂, 2-furonitrile, and a solvent to produce a carbonic acid ester.

In this step, water is also produced in addition to the carbonic acid ester as a result of the reaction between an alcohol and carbon dioxide. However, since 2-furonitrile is present, 2-furamide is produced by a hydration reaction with the produced water. Further, by removing the produced water from a reaction system or reducing the amount of the produced water in a reaction system, the production of a carbonic acid ester can be promoted. An example of this step is shown by the following formula.

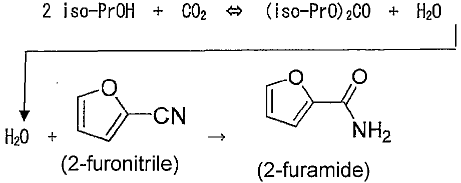

### (Alcohol)

The alcohol to be used may be any one or more selected from a primary alcohol, a secondary alcohol, and a tertiary alcohol. For example, the use of methanol, ethanol, 1-propanol, isopropanol, 1-butanol, 1-pentanol, 1-hexanol, 1-heptanol, 1-octanol, 1-nonanol, allyl alcohol, 2-methyl-1-propanol, cyclohexanemethanol, benzyl alcohol, ethylene glycol, 1,2-propanediol, or 1,3-propanediol is preferred because a product can be obtained in a high yield and a high reaction speed is achieved. Carbonic acid esters produced in this case are respectively dimethyl carbonate, diethyl carbonate, dipropyl carbonate, diisopropyl carbonate, dibutyl carbonate, dipentyl carbonate, dihexyl carbonate, diheptyl carbonate, dioctyl carbonate, dinonane carbonate, diallyl carbonate, di-2-methyl-propyl carbonate, dicyclohexanemethyl carbonate, dibenzyl carbonate, ethylene carbonate, 1,2-propylene carbonate, and 1,3-propylene carbonate.

When a carbonic acid ester to be obtained is used as a raw material of a diaryl carbonate, the alcohol to be used is preferably an alcohol having 1 to 6 carbon atoms, more preferably an alcohol having 2 to 4 carbon atoms.

Further, a monovalent or divalent alcohol is preferably used.

### (Catalyst for producing carbonic acid ester)

In the first reaction step to produce a carbonic acid ester, one or both of CeO₂ and ZrO₂ as solid catalysts are preferably used. For example, only CeO₂, only ZrO₂, a mixture of CeO₂ and ZrO₂, or a CeO₂-ZrO₂ solid solution or composite oxide is preferred, and only CeO₂ is particularly preferably used. In the case of a CeO₂-ZrO₂ solid solution or composite oxide, the mixing ratio between CeO₂ and ZrO₂ is basically 50:50 but may appropriately be changed.

The solid catalyst used in the first reaction step may be in the form of either powder or a shaped form. In the case of a shaped form, the solid catalyst may have any shape such as a spherical shape, a pellet-like shape, a cylindrical shape, a ring shape, a wheel-like shape, or a granular shape.

### (Carbon dioxide)

Carbon dioxide used in the present invention may be not only one prepared as an industrial gas but also one separated and recovered from emission gases from plants to produce various products, steel plants, electric power plants, or the like.

### (Solvent in carbonic acid ester production reaction)

In the carbonic acid ester production reaction, a solvent is preferably used which has a boiling point higher than that of 2-furamide to be produced. More preferably, the solvent used in the carbonic acid ester production reaction includes at least one selected from dialkylbenzene, alkylnaphthalene, and diphenylbenzene, and specific examples of such a solvent include Barrel Process Oil B28AN and Barrel Process Oil B30 (manufactured by Matsumura Oil Co., Ltd.), each of which contains components such as dialkylbenzene, alkylnaphthalene, and diphenylbenzene.

### (Reaction liquid temperature)

The temperature of a reaction liquid in the carbonic acid ester production reaction is preferably 50 to 300°C. If the temperature of the reaction liquid is lower than 50°C, both of the carbonic acid ester synthesis reaction and the hydration reaction of 2-furonitrile hardly proceed due to their low reaction speeds, and therefore the productivity of a carbonic acid ester tends to be low. On the other hand, if the temperature of the reaction liquid exceeds 300°C, the reaction speed of each of the reactions is high, but decomposition or denaturation of a carbonic acid ester is likely to occur and 2-furamide is likely to react with the alcohol, and therefore the yield of a carbonic acid ester tends to be low. The temperature of the reaction liquid is more preferably 100 to 150°C. However, such a preferred temperature of the reaction liquid is considered to vary depending on the type or amount of the solid catalyst or the amounts of or the ratio between the raw materials (alcohol and 2-furonitrile), and therefore it is desirable that an optimum condition is appropriately set. Since the preferred temperature of the reaction liquid is 100 to 150°C, it is desirable that the raw materials (alcohol and 2-furonitrile) are pre-heated with steam or the like at a stage before a carbonic acid ester reactor.

### (Reaction pressure)

The reaction pressure of the carbonic acid ester production reaction is preferably 0.1 to 20 MPa (absolute pressure). If the reaction pressure is less than 0.1 MPa (absolute pressure), a decompressor is required, which complicates facilities and increases costs. In addition to that, power and energy to reduce the pressure are required so that energy efficiency reduces. On the other hand, if the reaction pressure exceeds 20 MPa, the hydration reaction of 2-furonitrile is less likely to proceed so that the yield of a carbonic acid ester reduces. In addition to that, power and energy to increase the pressure are required so that energy efficiency reduces. From the viewpoint of increasing the yield of a carbonic acid ester, the reaction pressure is more preferably 0.5 to 15 MPa (absolute pressure), even more preferably 1.0 to 10 MPa (absolute pressure).

### (Amount of 2-furonitrile to be used)

It is desirable that 2-furonitrile used in the hydration reaction is previously introduced into a reactor before the reaction in a molar quantity that is 0.2 times or more and 5 times or less the theoretical molar quantity of water generated as a by-product by the reaction between an alcohol and CO₂ as raw materials. The molar quantity of 2-furonitrile is more desirably 0.5 times or more and 3 times or less, particularly desirably 0.8 times or more and 1.5 times or less the theoretical molar quantity of water generated as a by-product by the reaction between an alcohol and CO₂ as raw materials. If the molar quantity of 2-furonitrile is too small, there is a fear that the amount of 2-furonitrile that contributes to the hydration reaction is small so that the yield of a carbonic acid ester reduces. On the other hand, if 2-furonitrile is introduced in a molar quantity excessively large with respect to the alcohol as a raw material, the side reaction of 2-furonitrile undesirably increases. Further, the amounts of the alcohol and 2-furonitrile with respect to the solid catalyst are considered to vary depending on the type or amount of the solid catalyst, the type of the alcohol, or the ratio between the alcohol and 2-furonitrile, and therefore it is desirable that optimum conditions are appropriately set.

### (Separation by distillation)

After the reaction, a carbonic acid ester as a principal product, 2-furamide as a by-product, unreacted 2-furonitrile, and the solid catalyst such as CeO₂ are separated by distillation. As a result, the product can be collected.

### (Second reaction step)

Then, in a second reaction step in the present invention, 2-furamide generated as a by-product in the first reaction step is separated from the system after the carbonic acid ester production reaction and is then subjected to a dehydration reaction to produce 2-furonitrile. The second reaction step corresponds to the above-described method for producing 2-furonitrile, and therefore detailed description thereof is not repeated.

### (Reuse of 2-furonitrile)

2-furonitrile regenerated in the second reaction step can be reused in the first reaction step (hydration reaction).

### Examples

Hereinbelow, the present invention will be described in more detail with reference to examples, but the present invention is not limited to these examples.

### <Gas chromatographic (GC) analysis of reaction product>

Each reaction product obtained was dissolved in 20 g of acetone, and the resultant was passed through a filter, placed in a vial for GC analysis, and subjected to GC analysis.

### (GC and measurement conditions)

GC column: CP-Sil 5 CB (length 50 m, membrane thickness 0.25 µm, inner diameter 0.25 mm) manufactured by Agilent Technologies Inc.
Inlet pressure: 100 kPa
Vaporizing chamber temperature: 320°C
Column flow rate: 0.83 mL/min
Linear velocity: 19.9 cm/s
Sprit ratio: 25.0
Total flow rate: 24.5 mL/min
Carrier gas: N₂
Detector: Hydrogen flame ionization detector (FID)
Detector temperature: 320°C
Internal standard substance: Dodecane (manufactured by FUJIFILM Wako Pure Chemical Corporation, purity 97%)
Column temperature rise program:
   (Initial stage) temperature 50°C, retention time 0 min
   (First stage) temperature rise rate 30°C/min, achieving temperature 130°C, retention time 0 min
   (Second stage) temperature rise rate 5°C/min, achieving temperature 180°C, retention time 0 min
   (Third stage) temperature rise rate 30°C/min, achieving temperature 300°C, retention time 10 min

### (Preparation Example 1 of supported catalyst)

On the basis of an incipient-wetness method, a supported catalyst was prepared according to the following procedure.

In a 5-mL vial, 0.089 g of (NH₄)6Mo₇O₂₄·4H₂O (assay as MoO₃ 81%, manufactured by Wako Pure Chemical Industries, Ltd., special grade chemical, distributor 010-06905, Lot No. PTN1484) was precisely weighed and diluted with 3 mL of distilled water to prepare a catalyst precursor solution.

The catalyst precursor solution was dropped little by little into a 100-mL beaker containing 0.96 g of SiO₂ while the resultant was heated and stirred. The resultant was continued to be heated on a hot stirrer to evaporate moisture, and was then dried in an oven at 110°C for 12 hours. The obtained catalyst was placed in a muffle furnace and heated to 500°C at a temperature rise rate of 10°C/min and then continuously fired at 500°C for 3 hours to obtain a SiO₂-supported Mo catalyst with a Mo loading of 0.5 mmol/g.

### (Preparation Example 2 of supported catalyst)

A SiO₂-supported Cs catalyst with a Cs loading of 0.5 mmol/g was obtained in the same manner as in Preparation Example 1 except that 0.081 g of cesium carbonate (anhydrous) (assay: 95.0 to 102.0% (titration), manufactured by FUJIFILM Wako Pure Chemical Corporation, Wako 1^{st} Grade, distributor 034-06542, Lot No. CAG5233) was used instead of (NH₄)6Mo₇O₂₄·4H₂O.

### <Example 1>

### (Dehydration reaction of 2-furamide)

In a reaction tube containing a stirrer, 340 mg (3 mmol) of 2-furamide (manufactured by AK Scientific, Inc., purity 98.0%) as a raw material, 400 mg of the SiO₂-supported Mo catalyst obtained in Preparation Example 1, 0.1 g of dodecane (manufactured by FUJIFILM Wako Pure Chemical Corporation) as an internal standard substance, 20 g of mesitylene as a solvent, a net, wool, and 2 g of molecular sieve 3A previously fired at 300°C for 1 hour as a desiccant were placed in order. The reaction tube was set in a reactor (furnace) shown in Figure 1 having a cooler located above a joint part and a balloon located above the cooler.

The reactor (furnace) was set at 180°C and 600 rpm, and a reaction solution was subjected to reaction for 72 hours after the reaction solution boiled. The time when boiling of the reaction solution was identified was defined as a reaction start time. The reaction solution was taken out of the reactor and cooled to obtain a reaction product. The time when the reaction solution was taken out of the reactor was defined as a reaction stop time. The yield of the obtained 2-furonitrile was 91%.

It should be noted that the yield (%) of 2-furonitrile was calculated from the amount of 2-furamide by the following calculation formula. Yield (%) of 2-furonitrile = amount of 2-furonitrile obtained [mmol] / amount of 2-furamide as raw material [mmol] × 100

### <Example 2>

A reaction product was obtained in the same manner as in Example 1 except that 680 mg (6.0 mmol) of 2-furamide (manufactured by AK Scientific, Inc., purity 98.0%) was used as a raw material. The yield of the obtained 2-furonitrile was 88%.

### <Example 3>

A reaction product was obtained in the same manner as in Example 1 except that the desiccant (molecular sieve) was not used. The yield of the obtained 2-furonitrile was 86%.

### <Example 4>

A reaction product was obtained in the same manner as in Example 1 except that the amount of the catalyst added was changed from 400 mg to 100 mg and that the reaction time was changed from 72 hours to 24 hours. The yield of the obtained 2-furonitrile was 57%.

### <Example 5>

A reaction product was obtained in the same manner as in Example 4 except that the desiccant (molecular sieve) was not used. The yield of the obtained 2-furonitrile was 53%.

### <Example 6>

A reaction product was obtained in the same manner as in Example 1 except that the reaction time was changed from 72 hours to 6 hours. The yield of the obtained 2-furonitrile was 25%.

### <Example 7>

A reaction product was obtained in the same manner as in Example 6 except that the desiccant (molecular sieve) was not used. The yield of the obtained 2-furonitrile was 17%.

### <Comparative Example 1>

A reaction product was obtained in the same manner as in Example 4 except that the SiO₂-supported Cs catalyst obtained in Preparation Example 2 was used as a catalyst. The yield of the obtained 2-furonitrile was 38%.

### <Comparative Example 2>

A reaction product was obtained in the same manner as in Example 5 except that the SiO₂-supported Cs catalyst obtained in Preparation Example 2 was used as a catalyst. The yield of the obtained 2-furonitrile was 27%.

### <Comparative Example 3>

A reaction product was obtained in the same manner as in Example 6 except that the SiO₂-supported Cs catalyst obtained in Preparation Example 2 was used as a catalyst. The yield of the obtained 2-furonitrile was 14%.

### <Comparative Example 4>

A reaction product was obtained in the same manner as in Example 7 except that the SiO₂-supported Cs catalyst obtained in Preparation Example 2 was used as a catalyst. The yield of the obtained 2-furonitrile was 16%.

**[Table 1]**

| | Catalyst | | Desiccant | | Substrate (raw material) 2-furamide [mmol] | Amount of obtained product [mmol] | | | Residual substrate | |
|---|---|---|---|---|---|---|---|---|---|---|
| | Type of catalyst | Amount of catalyst added [mg] | Molecular sieve | Reaction time [hr] | | 2-furonitrile | 2-furancarboxylic acid | Others | 2-furamide [mmol] | Yield of 2-furonitrile [%] |
| Example 1 | Mo/SiO₂ | 400 | Present | 72 | 3.0 | 2.7 | 0.1 | <0.1 | 0.0 | 91 |
| Example 2 | Mo/SiO₂ | 400 | Present | 72 | 6.0 | 5.3 | 0.2 | <0.1 | 0.1 | 88 |
| Example 3 | Mo/SiO₂ | 400 | Absent | 72 | 3.0 | 2.6 | 0.1 | <0.1 | 0.2 | 86 |
| Example 4 | Mo/SiO₂ | 100 | Present | 24 | 3.0 | 1.7 | 0.2 | <0.1 | 1.0 | 57 |
| Example 5 | Mo/SiO₂ | 100 | Absent | 24 | 3.0 | 1.6 | 0.0 | <0.1 | 1.2 | 53 |
| Comparative Example 1 | Cs/SiO₂ | 100 | Present | 24 | 3.0 | 1.2 | 0.2 | <0.1 | 1.5 | 38 |
| Comparative Example 2 | Cs/SiO₂ | 100 | Absent | 24 | 3.0 | 0.8 | <0.1 | <0.1 | 2.2 | 27 |
| Example 6 | Mo/SiO₂ | 100 | Present | 6 | 3.0 | 0.7 | <0.1 | <0.1 | 1.0 | 25 |
| Example 7 | Mo/SiO₂ | 100 | Absent | 6 | 3.0 | 0.5 | 0.0 | <0.1 | 1.2 | 17 |
| Comparative Example 3 | Cs/SiO₂ | 100 | Present | 6 | 3.0 | 0.4 | <0.1 | <0.1 | 2.2 | 14 |
| Comparative Example 4 | Cs/SiO₂ | 100 | Absent | 6 | 3.0 | 0.5 | <0.1 | <0.1 | 2.2 | 16 |

### <Example 8>

Cerium oxide (manufactured by Solvay Special Chem Japan, Ltd., HSA-20SP, average particle diameter about 10 µm, impurity concentration 0.02% or less) was fired in an air atmosphere at 600°C for 3 hours to obtain a powdery solid catalyst.

Then, 0.344 g (2.0 mmol) of the solid catalyst, 2.33 g (25.0 mmol) of 2-furonitrile obtained in Example 1, and 0.751 g of 1-propanol (12.5 mmol, manufactured by FUJIFILM Wako Pure Chemical Corporation) were placed in an autoclave (with two inclined stirring paddles, material SUS316, capacity 190 mL). After purging with CO₂, the system was filled with CO₂ to increase the pressure to 5 MPa to perform a reaction (i.e., an aliphatic carbonic acid ester production reaction) at a reaction temperature of 132°C for 4 hours.

Then, the autoclave was cooled and depressurized to collect a reaction liquid. The reaction liquid was analyzed by gas chromatography. As a result, 7.31 g (5.0 mmol) of dipropyl carbonate (DPrC) as a target product and 0.67 g (6.0 mmol) of 2-furamide, 0.0412 g (0.04 mmol) of propyl carbamate, 0.0154 g (0.10 mmol) of propyl furan-2-carboxylate, and 0.0153 g (0.10 mmol) of propyl furan-2-imidate as byproducts were detected.

Then, the 2-furamide obtained as a by-product was separated, and then the same operation as in Example 1 was performed using the 2-furamide to regenerate 2-furonitrile. Then, the regenerated 2-furonitrile was reused in the aliphatic carbonic acid ester production reaction.

## Claims

1. A method for producing 2-furonitrile, comprising dehydrating 2-furamide in the presence of an Mo/SiO₂ catalyst in which molybdenum (Mo) is supported on a carrier formed from SiO₂.

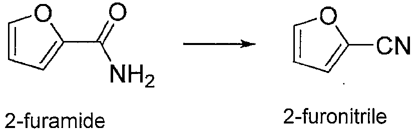

2. The method for producing 2-furonitrile according to claim 1, wherein the dehydration is further performed in the presence of a desiccant.

3. The method for producing 2-furonitrile according to claim 2, wherein the desiccant is a molecular sieve.

4. The method for producing 2-furonitrile according to any one of claims 1 to 3, wherein 2-furancarboxylic acid is produced together with the 2-furonitrile.

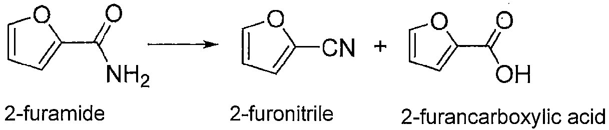

5. A method for producing a carbonic acid ester, comprising:
a first reaction step including a carbonic acid ester production reaction in which an alcohol and carbon dioxide are reacted in the presence of a solid catalyst, 2-furonitrile, and a solvent to produce a carbonic acid ester and water and a hydration reaction in which the 2-furonitrile is hydrated with the produced water to produce 2-furamide; and
a second reaction step in which the 2-furamide is separated from a reaction system of the first reaction step, and then dehydration of the 2-furamide is performed in the presence of an Mo/SiO₂ catalyst in which molybdenum (Mo) is supported on a carrier formed from SiO₂ to regenerate 2-furonitrile, wherein
at least a part of the 2-furonitrile regenerated in the second reaction step is used in the first reaction step.

6. The method for producing a carbonic acid ester according to claim 5, wherein the dehydration is further performed in the presence of a desiccant.

7. The method for producing a carbonic acid ester according to claim 6, wherein the desiccant is a molecular sieve.

8. The method for producing a carbonic acid ester according to any one of claims 5 to 7, wherein the alcohol comprises an alcohol having 1 to 6 carbon atoms.

9. The method for producing a carbonic acid ester according to any one of claims 5 to 8, wherein the solid catalyst comprises at least one selected from CeO₂ and ZrO₂.

10. The method for producing a carbonic acid ester according to any one of claims 5 to 9, wherein in the first reaction step, a solvent having a boiling point higher than that of the 2-furamide to be produced is used.

11. The method for producing a carbonic acid ester according to claim 10, wherein the solvent comprises at least one selected from dialkylbenzene, alkylnaphthalene, and diphenylbenzene.

## Patentansprüche

1. Verfahren zur Herstellung von 2-Furonitril, umfassend die Dehydratisierung von 2-Furamid in Gegenwart eines Mo/SiO₂-Katalysators, in dem Molybdän (Mo) geträgert ist auf einem aus SiO2 gebildeten Träger.

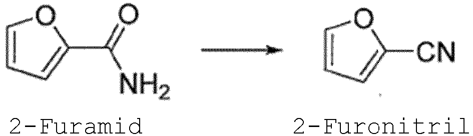

2. Verfahren zur Herstellung von 2-Furonitril gemäß Anspruch 1, wobei die Dehydratisierung ferner in Gegenwart eines Trocknungsmittels durchgeführt wird.

3. Verfahren zur Herstellung von 2-Furonitril gemäß Anspruch 2, wobei das Trocknungsmittel ein Molekularsieb ist.

4. Verfahren zur Herstellung von 2-Furonitril gemäß mindestens einem der Ansprüche 1 bis 3, wobei 2-Furancarbonsäure zusammen mit dem 2-Furonitril hergestellt wird.

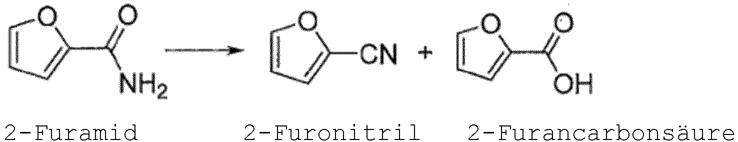

5. Verfahren zur Herstellung eines Kohlensäureesters, umfassend:
einen ersten Reaktionsschritt, enthaltend eine Kohlensäureester-Herstellungsreaktion, bei der ein Alkohol und Kohlendioxid in Gegenwart eines festen Katalysators, 2-Furonitril und eines Lösungsmittels umgesetzt werden, um herzustellen einen Kohlensäureester und Wasser und eine Hydratisierungsreaktion, bei der das 2-Furonitril mit dem hergestellten Wasser hydratisiert wird, um 2-Furamid herzustellen; und
einen zweiten Reaktionsschritt, in dem das 2-Furamid aus einem Reaktionssystem des ersten Reaktionsschritts abgetrennt wird, und anschließend Dehydratisierung des 2-Furamids in Gegenwart eines Mo/SiO₂-Katalysators durchgeführt wird, in dem Molybdän (Mo) auf einem aus SiO₂ gebildeten Träger geträgert ist, um 2-Furonitril zu regenerieren, wobei
mindestens ein Teil des im zweiten Reaktionsschritt regenerierten 2-Furonitrils im ersten Reaktionsschritt verwendet wird.

6. Verfahren zur Herstellung eines Kohlensäureesters gemäß Anspruch 5, wobei die Dehydratisierung ferner in Gegenwart eines Trocknungsmittels durchgeführt wird.

7. Verfahren zur Herstellung eines Kohlensäureesters gemäß Anspruch 6, wobei das Trocknungsmittel ein Molekularsieb ist.

8. Verfahren zur Herstellung eines Kohlensäureesters gemäß mindestens einem der Ansprüche 5 bis 7, wobei der Alkohol einen Alkohol mit 1 bis 6 Kohlenstoffatomen umfasst.

9. Verfahren zur Herstellung eines Kohlensäureesters gemäß mindestens einem der Ansprüche 5 bis 8, wobei der feste Katalysator mindestens einen, ausgewählt aus CeO₂ und ZrO₂, umfasst.

10. Verfahren zur Herstellung eines Kohlensäureesters gemäß mindestens einem der Ansprüche 5 bis 9, wobei in dem ersten Reaktionsschritt ein Lösungsmittel mit einem höheren Siedepunkt als dem des herzustellenden 2-Furamids verwendet wird.

11. Verfahren zur Herstellung eines Kohlensäureesters gemäß Anspruch 10, wobei das Lösungsmittel mindestens eines ausgewählt aus Dialkylbenzol, Alkylnaphthalin und Diphenylbenzol umfasst.

## Revendications

1. Un procédé de production de 2-furonitrile, comprenant la déshydratation de 2-furamide en présence d'un catalyseur Mo/SiO2 dans lequel du molybdène (Mo) est supporté sur un support formé à partir de SiO₂.

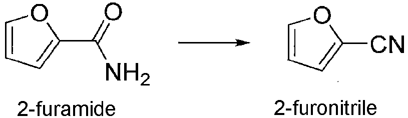

2. Le procédé de production de 2-furonitrile selon la revendication 1, dans lequel la déshydratation est en outre effectuée en présence d'un dessiccant.

3. Le procédé de production de 2-furonitrile selon la revendication 2, dans lequel le dessiccant est un tamis moléculaire.

4. Le procédé de production de 2-furonitrile selon l'une quelconque des revendications 1 à 3, dans lequel l'acide 2-furancarboxylique est produit en même temps que le 2-furonitrile.

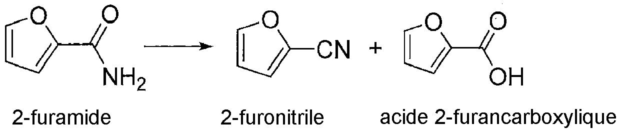

5. Un procédé de production d'un ester d'acide carbonique, comprenant :
une première étape de réaction comprenant une réaction de production d'ester d'acide carbonique dans laquelle un alcool et du dioxyde de carbone sont mis à réagir en présence d'un catalyseur solide, de 2-furonitrile et d'un solvant pour produire un ester d'acide carbonique et de l'eau, et une réaction d'hydratation dans laquelle le 2-furonitrile est hydraté avec l'eau produite pour produire du 2-furamide ; et
une deuxième étape de réaction dans laquelle le 2-furamide est séparé d'un système réactionnel de la première étape de réaction, puis une déshydratation du 2-furamide est effectuée en présence d'un catalyseur Mo/SiO₂ dans lequel du molybdène (Mo) est supporté sur un support formé à partir de SiO₂ pour régénérer le 2-furonitrile, dans lequel
au moins une partie du 2-furonitrile régénéré dans la deuxième étape de réaction est utilisée dans la première étape de réaction.

6. Le procédé de production d'un ester d'acide carbonique selon la revendication 5, dans lequel la déshydratation est en outre effectuée en présence d'un dessiccant.

7. Le procédé de production d'un ester d'acide carbonique selon la revendication 6, dans lequel le dessiccant est un tamis moléculaire.

8. Le procédé de production d'un ester d'acide carbonique selon l'une quelconque des revendications 5 à 7, dans lequel l'alcool comprend un alcool ayant 1 à 6 atomes de carbone.

9. Le procédé de production d'un ester d'acide carbonique selon l'une quelconque des revendications 5 à 8, dans lequel le catalyseur solide comprend au moins un élément choisi parmi CeO₂ et ZrO₂.

10. Le procédé de production d'un ester d'acide carbonique selon l'une quelconque des revendications 5 à 9, dans lequel, dans la première étape de réaction, on utilise un solvant ayant un point d'ébullition supérieur à celui du 2-furamide à produire.

11. Le procédé de production d'un ester d'acide carbonique selon la revendication 10, dans lequel le solvant comprend au moins un composé choisi parmi le dialkylbenzène, l'alkylnaphtalène et le diphénylbenzène.
